# EUROPEAN PATENT SPECIFICATION

(11) **EP 2 754 432 B1**
(45) Date of publication and mention of the grant of the patent: **17.10.2018**
(21) Application number: 14150832.5
(22) Date of filing: 10.01.2014
(51) Int. Cl.: A61K 8/26, A61K 8/81, A61K 8/85, A61K 8/02, A61Q 19/00, A61Q 19/08, A61K 8/89

(54) **Aqueous compositions comprising polymer particles and low levels of clay**
Wässrige Zusammensetzungen mit Polymerpartikeln und niedrigem Tongehalt
Compositions aqueuses comprenant des particules de polymère et de faibles niveaux d'argile

(30) Priority: 10.01.2013 US 201313738001
(43) Date of publication of application: 16.07.2014
(73) Proprietor: Johnson & Johnson Consumer Inc., Skillman, NJ 08558 (US)
(72) Inventor: Chen, Theresa, Princeton, NJ New Jersey 08540 (US); Lee, Christina Irene, Plainsboro, NJ New Jersey 08536 (US); Thakrar, Jipsha, Westampton, NJ New Jersey 08060 (US); Brillouet, Anne-Sophie, Pennington, NJ New Jersey 08534 (US); Dufort, Marisa DeVita, Hillsborough, NJ New Jersey 08844 (US)
(74) Representative: Kirsch, Susan Edith

(56) References cited:
- KR-A- 20110 073 935
- US-A1- 2004 191 283
- US-A1- 2005 169 867
- US-A1- 2009 263 660
- DATABASE GNPD [Online] MINTEL; August 2012 (2012-08), "oil control foundation primer", XP002742893, Database accession no. 1866180
- DATABASE GNPD [Online] MINTEL; December 2007 (2007-12), "deep wrinkle concentrate", XP002742894, Database accession no. 832473
- DATABASE GNPD [Online] MINTEL; September 2011 (2011-09), "ultra anti-aging serum", XP002742895, Database accession no. 16244474

## Description

### FIELD OF THE INVENTION

The present invention relates to a composition comprising polymer particles, a clay and methods of use thereof.

### BACKGROUND OF THE INVENTION

Wrinkles, puffiness, and sagging, particularly related to skin under the eyes are problematic signs of aging. While it is known to apply various compositions to skin under the eyes, various drawbacks still remain. In particular, the inventors have recognized that it would be desirable to reduce the appearance of the above signs of under eye skin aging in a manner that provides a pleasant tightening sensation to skin under the eyes. Even more importantly, the inventors have recognized that it is important to provide these benefits without introducing negative perceptions, such as *over*-tightening or undesirable flaking of the composition.

Accordingly, the inventors have now discovered that a particular composition that includes 0.75% to 1.25% by weight of a clay portion comprising bentonite and polymer particles. The polymer particles have an average particle size of less than 20 microns and a refractive index of 3 to 1.4. The composition is completely free of hydrophobic compounds. The composition provides an improved appearance in a comfortable manner with reduced flaking.

US 2005/169867 discloses treating the surface of particles of at least one powder selected from PMMA, nylon, polystyrene, a silicone elastomer powder, benzoguanamine, a styrenedivinylbenzene pinhole polymer, ethylene tetrafluoride, a polyethylene powder, a polyurethane powder, a silk powder, an organic pigment and a composite of at least one of these and a metal oxide and/or a metal hydroxide with a low-molecular organosilicon derivative, with or without a water-soluble cationic polymer.

US 2004/191283 discloses a texturizing composition and method utilized for foundations, makeup powders, mascaras, lipsticks, emulsions, lotions, gels, sprayable formulations, and solutions impregnated on fabrics, paper, towelettes or complexion corrector papers comprising at least one self-invertible inverse latex, and powder.

US 2009/263660 discloses that by coating the surface of a powder comprising a silicone resin and/or an organic powder with a specific hydrophilizing agent, such powder is hydrophilized.

KR 2011 0073935 discloses a cosmetic composition containing adipic acid/neopentyl glycol crosspolymer suspension solution.

"Oil Control Foundation Primer", DATABASE GNPD, MINTEL, (201208), Database accession no. 1866180, XP002742893, discloses a composition containing AuraSphere® N particles.

"Deep Wrinkle Concentrate", DATABASE GNPD, MINTEL, (200712), Database accession no. 832473, XP002742894, discloses a composition containing AuraSphere® N particles.

"Ultra Anti-Aging Serum", DATABASE GNPD, MINTEL, (201109), Database accession no. 16244474, XP002742895, discloses a composition containing AuraSphere® N particles.

### SUMMARY OF THE INVENTION

The present invention features a composition that comprises 0.75% to 1.25% by weight of a clay portion, wherein the clay portion comprises bentonite. The composition further comprises polymer particles that have an average particle size of less than 20 microns and a refractive index of 1.3 to 1.4, wherein the polymer particles include a crosslinked polyester that is a mixture of a polyester of adipic acid and neopentyl glycol crosslinked with isopropyltriethylsilane, blended with a copolymer of vinyl pyrrolidine and vinyl acetate. The composition is completely free of hydrophobic compounds. The composition is particularly useful for treating skin under the eyes.

### DETAILED DESCRIPTION OF THE INVENTION

Unless defined otherwise, all technical and scientific terms used herein have the meaning commonly understood by one of ordinary skill in the art to which the invention pertains. Unless otherwise indicated, a percentage refers to a percentage by weight (i.e., %(W/W)).

As used herein, "signs of skin aging" means the presence of lines and wrinkles. As used herein, "wrinkle" means fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "substantially free" means less than about 2% by weight, or less than about 1.5% by weight, such as less than about 1% by weight of the ingredient to which it refers.

As used herein, the term "comprising" encompasses "including" as well as "consisting" and "consisting essentially of' e.g. a composition comprising X may consist exclusively of X or may include something additional e.g. X + Y.

It is believed that one skilled in the art can, based upon the description herein, utilize the present invention to its fullest extent. The scope of the invention is defined by the appended claims.

### Clay Portion

The clay portion of the composition comprises one or more clays. "Clay," as used herein, refers to a class of hydrous silicate minerals that may finely divide in water so as to have an average (e.g., median) particle size of about 5 microns or less, such as about 2 microns or less. Clays are typically water insoluble, but are readily dispersible in water. Chemically, clays typically include silicon and oxygen as well as chemically bound water (hydrogen and oxygen), and may also include aluminum, magnesium, lithium, calcium, among other chemical moieties.

Clays suitable for use in compositions of the present invention may be single minerals or blends thereof, but the clay includes at least one bentonite.

"Bentonite" is an aluminum phyllosilicate that typically consists predominantly of montmorillonite and, as such, generally includes about 15% Al₂O₃ or more in its composition. In bentonite, the relative concentrations of sodium, potassium, calcium, as well as iron, magnesium and other elements may vary. The pH of bentonite in a 5% dispersion in deionized water may be from about 9 to about 10. One particularly suitable bentonite is VEEGUM HS, a high purity magnesium aluminum silicate commercially available from R.T. Vanderbilt of Norwalk, Connecticut.

It may be desirable that the clay portion comprises 20 percent bentonite or more. The clay portion may consist essentially of bentonite. Alternatively, the clay portion may consist of bentonite.

For example, the amount of bentonite in the clay portion may be greater than 95% percent.

It may be desirable to include, in addition to bentonite, a second clay, in particular a synthetic hectorite clay. Synthetic hectorite clays may include lithium (e.g., about 0.5% or more of LiO₂) and may further include magnesium and sodium in their chemical structure. Synthetic hectorite clays are typically capable of achieving lower average particle sizes in solution than bentonite, (e.g., less than 1 micron) and are further capable of achieving disk-like shapes in water. One particularly suitable synthetic hecrorite is LAPONITE XLG, a lithium magnesium sodium silicate available from Southern Clay Products of Gonzales, Texas. The clay portion may include 70 to 80% by weight synthetic hectorite and 20 to 30% by weight bentonite.

The inventors have surprisingly found that by using a small amount of clay portion, the composition provides the critical balance of little to no flaking with a pleasant tightening sensation to the user.

The clay portion comprises 0.75 to 1.25% by weight of the composition, such as 1% by weight of the composition.

### Polymer Particles

The composition includes polymer particles. Polymer particles suitable in the present invention are dispersible in water, with or without the assistance of a dispersing agent. The polymer(s) that comprise the polymer portion may have repeat units that include at least carbon and hydrogen, and optionally one or more of oxygen, silicon, nitrogen. Such polymer may comprise a polyester or acrylic monomer. The polymer comprising the polymer may be crosslinked. The polymer particles may be substantially spherical.

The inventors have found that polymer particles suitable in the present invention have a refractive index (such as may be measured at a wavelength of light from about 450nm to about 500nm) of 1.3 to 1.4, such as 1.30 to 1.38.

Furthermore, the polymer particles have an average particle size (e.g., median particle size or median particle diameter) that is less than 20 microns, say 1 to 20 microns. The average particle size of the polymer particles may be less than 10 microns or less than 8 microns. At least 90% of the polymer particles may have a particle size in a range of 0.5 to 10 microns. Less than 5% of the polymer particles may have a size greater than 26 microns.

The polymer particles includes a crosslinked polyester. The crosslinked polyester is a mixture of a polyester of adipic acid and neopentyl glycol crosslinked with isopropyltriethylsilane blended with a copolymer of vinyl pyrrolidone and vinyl acetate and is commercially available as AURASPHERE N (containing about 50% total polymer) from Centerchem, Inc. of Norwalk, Connecticut. The polymer particles of AURASPHERE N have a refractive index of 1.35 and a median particle size from about 0.5 to about 6 microns.

The amount of polymer particles in the composition is desirably 1% to 20%, such as 2% to 12%, such as 2% to 10%, such as 2% to 8%, for example 2% to 5%, by weight of the composition.

Furthermore, the weight ratio of polymer particles to clay portion (total amount of clays) may be 1:1 to 10:1, such as 2:1 to 8:1, or 2.5:1 to 5:1.

The compositions of the present invention provide a balanced combination of pleasing sensations, both tactile and visual.

### Additional Polymers

The composition may further include additional polymers such as those suitable for one or more of: enhancing the dispersion of the polymer particles in the composition, providing a gelling or thickening effect, or providing/enhancing film-formation. Examples of suitable additional polymers include synthetic polymers, in particular nonionic and/or cationic synthetic polymers. The additional polymer may have a weight average molecular weight of 1000 to 750,000.

Suitable synthetic polymers include homopolymers or copolymers such as those comprised of vinyl or acrylic monomers. Suitable vinyl polymers include copolymers of vinyl pyrrolidone, such as copolymers of vinyl pyrrolidone and vinyl acetate. Suitable acrylic polymers include polyacrylamides. An example of a suitable polyacrylamide is SEPIGEL 305, which includes about 45% polyacrylamide (and further includes laureth-7 and C13-C14 isoparaffin and water) and is commercially available from Seppic of Paris, France. Other suitable synthetic polymers are derivatized cellulose polymers. One suitable cellulose polymer is hydroxypropyl cellulose. Suitable cationic polymers include polymers derivatized with quaternium ammonium, e.g., polyquaternium-51.

While the composition may include natural polymers, the composition may be substantially free, or completely free, of polysaccharide polymers including: polysaccharide gums (e.g, natural polysaccharide gums such as xanthan gum, guar gum, carob gum and the like, such as those having a molecular weight of 100,000 or greater, such as those having a molecular weight of 200,000 or greater, such as those having a molecular weight 750,000 daltons or greater) and other natural or chemically modified polysaccharides or synthetic polysaccharides, such as chitosan, pectin, cationic and non-ionic cellulose polymers, and starches.

The composition may be substantially free of natural polysaccharides gum polymers (such as those defined above), but may include other polysaccharides.

Alternatively or in addition, the relative amount of polysaccharide polymers compared to the total amount of additional polymer in the composition may be minimized. For example, the total amount of polysaccharide polymers may be 10% by weight or less, say less than 5% by weight, of the total amount of additional polymers in the composition.

The total amount of additional polymers in the composition is desirably from 05% to 3%, such as from about 1% to 2%, by weight of the composition.

### Humectant

The composition may further include one or more humectants. Humectants are hygroscopic and capable of hydrogen bonding with water and, the humectant may include at least two or more of the following functional groups: hydroxyl groups, amine/amino groups, and/or carboxylic acid groups. The humectant has less than 3 carbon atoms per above functional group. Particularly suitable humectants include glycerin, butylene glycol, propylene glycol, urea, and trehalose. One notable ingredient that includes the humectants glycerin, sodium PCA (an amino acid) urea, and trehalose is ADVANCED MOISTURE COMPLEX available from BASF of Ludwigshafen, Germany.

The concentration of humectants may range from 3% to 20%, such as from 4% to 15%, such as from 6 to 15%, by weight of the composition.

Furthermore, the weight ratio of humectants to clay portion may be 5:1 to 20:1, such as 8:1 to 15:1.

### Topical Composition

The composition of the present invention, aside from including clays, polymers and humectants, further includes water. The amount of water in the composition may be 65% to 97%, such as 70% to 90%, such as 75% to 85%, by weight of the composition.
The composition is completely free of hydrophobic compounds. As used herein, a "hydrophobic compound" means a compound that includes a hydrophobic moiety meeting one or more of the following three criteria: (a) has a carbon chain of at least ten carbons in which none of the ten carbons is a carbonyl carbon or has a hydrophilic moiety (defined below) bonded directly to it; (b) has two or more, preferably five or more alkyl siloxy groups and is free of hydrophilic moieties; or (c) has two or more oxypropylene groups in sequence. The hydrophobic moiety may include linear, cyclic, aromatic, saturated or unsaturated groups. The hydrophobic compound is not amphiphilic and, and such, does not include hydrophilic moieties. Hydrophilic moieties include anionic, cationic, zwitterionic, or nonionic group, that is polar, including sulfate, sulfonate, carboxylate, phosphate, phosphonates, ammonium, including mono-, di-, and trialkylammonium species, pyridinium, imidazolinium, amidinium, poly(ethyleneiminium), ammonioalkylsulfonate, ammonioalkylcarboxylate, amphoacetate, amine, amino, and poly(ethyleneoxy)sulfonyl moieties.

Examples of hydrophobic compounds that the composition may be completely free of include C12 or greater oils such as vegetable oils (glyceryl esters of fatty acids, triglycerides), fatty esters, waxes, silicone oils, mineral oil and the like.

Compositions of the present invention may be substantially free of lower (e.g., C2-C3) monoalcohols such as ethanol and isopropanol.

Furthermore, compositions of the present invention may also be substantially free of color pigments, such as those typically used in color cosmetics (e.g., iron oxides, lake pigments, and interference pigments), to provide color to the skin.

Compositions of the present invention may otherwise include other functional ingredients such as anti-wrinkle agents, organic sunscreens, preservatives and fragrance. One example of a suitable preservative is EUXYL PE 9010, a mixture of phenoxyethanol and ethylhexylglycerin, available from Schulke & Mayr GmbH of Norderstedt, Germany.

The pH of the composition may be 6.5 or greater, such as 6.5 to 8.5, such as 7.5 to 8.5, more particularly 7.5 to 8.5.

Compositions of the present invention are particularly suitable for topically applying to skin, such as for treating signs of aging, and, in particular, to under eye skin (e.g., generally between the eyeballs and the upper part of the cheekbone) in order to reduce the appearance of wrinkles while maintaining a pleasant sensory experience. The composition may be contained within or be in fluid communication with an applicator that is suitable for dispensing the product directly to the under eye skin.

### Example 1

Four compositions (reference examples), compositions E1, E2, E3 and E4, were prepared. The ingredients are shown below in Table 1.

**TABLE 1**

| | | **Percent by Weight** | | | |
|---|---|---|---|---|---|
| **Ingredient** | **INCI** | **E1** | **E2** | **E3** | **E4** |
| Purified Water | Water | 73.2 | 78.2 | 78.2 | 73.2 |
| Laponite XLG | Lithium Magnesium Sodium Silicate | 0.75 | 0.75 | 0.75 | 0 |
| Veegum HS Granules | Magnesium Aluminum Silicate | 0.25 | 0.25 | 0.25 | 1.0 |
| Glycerin 99% USP | Glycerin | 2.0 | 2.0 | 2.0 | 2.0 |
| Advanced Moisture Complex | Glycerin, Sodium PCA, Urea, Polyquaternium 51; Sodium hyaluronate; Thehalose; Chlorphensin; Phenoxyethano 1; Methylparaben | 5.0 | 5.0 | 5.0 | 5.0 |
| Sepigel 305 | Polyacrylamide; Laureth-7; C13-C14 isoparaffin | 3.0 | 3.0 | 3.0 | 3.0 |
| 1,3 Butylene glycol | Butylene glycol | 5.0 | 5.0 | 5.0 | 5.0 |
| AuraSphere N | Polyester of adipic acid and neopentyl glycol crosslinked with isopropyltriethylsilane blended with a copolymer of vinyl pyrrolidone and vinyl acetate | 10.0 | 5.0 | | 10.0 |
| Sepimat CP5 | Methylmethacrylate crosspolymer | | | 5.0 | |
| Euxyl PE 9010 | Phenoxyethanol; Ethylhexyl glycerin | 0.8 | 0.8 | 0.8 | 0.8 |

### Example 2

Compositions E1 and E4 were evaluated in a consumer test along with a comparative composition C1, YOUTHOLOGY NINETY SECOND WRINKLE REMOVING EYE SERUM, commercially available from YOUTHOLOGY Research Institute of Pacoima, California. The compositions were tested by 240 women of varying ethnicities, ages 30-60, and having concerns about bags, puffiness, dark circles, lines and wrinkles. The products were evaluated for overall likeability at 5 minutes, 30 minutes, 3 hours, and 1 week after applying them to under eye skin.

The percentage of respondents placing compositions E1 and E4 into the negative categories of "dislike extremely," "dislike very much," "dislike moderately," or "dislike slightly" ranged from 3% to 12%. In contrast, the percentage of respondents placing composition C1 into the same negative categories was 20% to 24%. The difference in these percentage ranges was statistically significant.

According to its label, composition C1 did not contain polymer particles having an average particle size of less than about 20 microns and a refractive index from about 1.3 to about 1.4.

## Claims

1. A composition, comprising:
water;
0.75% to 1.25% by weight of a clay portion, wherein the clay portion comprises bentonite; and
polymer particles having an average particle size of less than 20 microns and a refractive index of 1.3 to 1.4, wherein the polymer particles include a crosslinked polyester that is a mixture of a polyester of adipic acid and neopentyl glycol crosslinked with isopropyltriethylsilane, blended with a copolymer of vinyl pyrrolidine and vinyl acetate;
wherein the composition is completely free of hydrophobic compounds;
wherein a hydrophobic compound is a compound that includes a hydrophobic moiety meeting one or more of the following criteria: (a) has a carbon chain of at least ten carbons in which none of the ten carbons is a carbonyl carbon or has a hydrophilic moiety bonded directly to it; (b) has two or more alkyl siloxy groups and is free of hydrophilic moieties; or (c) has two or more oxypropylene groups in sequence, wherein hydrophilic moieties include anionic, cationic or zwitterionic groups, or nonionic groups which are polar.

2. The composition of claim 1, wherein the clay portion comprises at least 20% by weight bentonite.

3. The composition of claim 1 or claim 2, wherein the clay portion further comprises synthetic hectorite.

4. The composition of any of the preceding claims wherein the clay portion comprises 70 to 80% by weight synthetic hectorite and 20 to 30% by weight bentonite.

5. The composition of any one of claims 1 to 3, wherein the amount of bentonite in the clay portion is greater than 95 % by weight.

6. The composition of claim 5, wherein the clay portion consists of bentonite.

7. The composition of any of the preceding claims comprising 2 to 8% by weight of the polymer particles.

8. The composition of any of the preceding claims further comprising an additional polymer having a weight average molecular weight of 1000 to 750,000.

9. The composition of any of the preceding claims, wherein the polymer particles have a refractive index of 1.3 to 1.38.

10. The composition of any of the preceding claims, comprising 70 to 90% by weight of water.

11. The composition of any of the preceding claims containing less than 2% by weight polysaccharide gums.

12. The composition of any of the preceding claims, wherein the weight ratio of the polymer particles to the clay portion is 2:1 to 8:1.

13. The composition of any of the preceding claims having a pH of 6.5 to 8.5.

14. The composition of any of the preceding claims having a pH of 7 to 8.

15. The composition of claim 1, comprising:
70% to 90% by weight of water;
0.75% to 1.25% by weight of a clay portion, wherein the clay portion comprises bentonite;
polymer particles having an average particle size of less than 20 microns and a refractive index of 1.3 to 1.4, wherein the polymer particles include a crosslinked polyester that is a mixture of a polyester of adipic acid and neopentyl glycol crosslinked with isopropyltriethylsilane, blended with a copolymer of vinyl pyrrolidine and vinyl acetate; and
an additional polymer having a weight average molecular weight of 1000 to 750,000.

16. Use of a composition for treating under eye skin under the eyes comprising topically applying the composition to said under eye skin, said composition comprising:
water;
0.75% to 1.25% by weight of a clay portion, wherein the clay portion comprises bentonite; and
polymer particles having an average particle size of less than 20 microns and a refractive index of 1.3 to 1.4, wherein the polymer particles include a crosslinked polyester that is a mixture of a polyester of adipic acid and neopentyl glycol crosslinked with isopropyltriethylsilane, blended with a copolymer of vinyl pyrrolidine and vinyl acetate;
wherein the composition is completely free of hydrophobic compounds;
wherein a hydrophobic compound is a compound that includes a hydrophobic moiety meeting one or more of the following criteria: (a) has a carbon chain of at least ten carbons in which none of the ten carbons is a carbonyl carbon or has a hydrophilic moiety bonded directly to it; (b) has two or more alkyl siloxy groups and is free of hydrophilic moieties; or (c) has two or more oxypropylene groups in sequence, wherein hydrophilic moieties include anionic, cationic or zwitterionic groups, or nonionic groups which are polar.

## Patentansprüche

1. Zusammensetzung, umfassend:
Wasser;
0,75 bis 1,25 Gew.-% eines Tonanteils, wobei der Tonanteil Bentonit umfasst; und
Polymerteilchen, die eine durchschnittliche Teilchengröße von weniger als 20 Mikron und einen Brechungsindex von 1,3 bis 1,4 aufweisen, wobei die Polymerteilchen einen vernetzten Polyester enthalten, der eine Mischung eines Polyesters von Adipinsäure und mit Isopropyltriethylsilan vernetztem Neopentylglycol, mit einem Copolymer von Vinylpyrrolidin und Vinylacetat gemischt, ist;
wobei die Zusammensetzung vollständig frei von hydrophoben Verbindungen ist;
wobei eine hydrophobe Verbindung eine Verbindung ist, die einen hydrophoben Anteil enthält, der einer oder mehr der folgenden Kriterien entspricht: (a) eine Kohlenstoffkette von mindestens zehn Kohlenstoffen aufweist, wobei keiner der zehn Kohlenstoffe ein Carbonylkohlenstoff ist oder einen hydrophilen, direkt daran gebundenen Anteil aufweist; (b) zwei oder mehr Alkylsiloxygruppen aufweist und von hydrophilen Anteilen frei ist; oder (c) zwei oder mehr Oxypropylengruppen hintereinander aufweist, wobei die hydrophilen Anteile anionische, kationische oder zwitterionische Gruppen oder nichtionische Gruppen enthält, die polar sind.

2. Zusammensetzung nach Anspruch 1, wobei der Tonanteil mindestens 20 Gew.-% Bentonit umfasst.

3. Zusammensetzung nach Anspruch 1 oder Anspruch 2, wobei der Tonanteil ferner synthetischen Hectorit umfasst.

4. Zusammensetzung nach irgendeinem der vorhergehenden Ansprüche, wobei der Tonanteil 70 bis 80 Gew.-% synthetischen Hectorit und 20 bis 30 Gew.-% Bentonit umfasst.

5. Zusammensetzung nach irgendeinem der Ansprüche 1 bis 3, wobei die Menge an Bentonit in dem Tonanteil mehr als 95 Gew.-% beträgt.

6. Zusammensetzung nach Anspruch 5, wobei der Tonanteil aus Bentonit besteht.

7. Zusammensetzung nach irgendeinem der vorhergehenden Ansprüche, umfassend 2 bis 8 Gew.-% der Polymerteilchen.

8. Zusammensetzung nach irgendeinem der vorhergehenden Ansprüche, ferner ein zusätzliches Polymer umfassend, das ein gewichtsdurchschnittliches Molekulargewicht von 1000 bis 750.000 aufweist.

9. Zusammensetzung nach irgendeinem der vorhergehenden Ansprüche, wobei die Polymerteilchen einen Brechungsindex von 1,3 bis 1,38 aufweisen.

10. Zusammensetzung nach irgendeinem der vorhergehenden Ansprüche, umfassend 70 bis 90 Gew.-% Wasser.

11. Zusammensetzung nach irgendeinem der vorhergehenden Ansprüche, enthaltend weniger als 2 Gew.-% Polysaccharidgummis.

12. Zusammensetzung nach irgendeinem der vorhergehenden Ansprüche, wobei das Gewichtsverhältnis der Polymerteilchen zu dem Tonanteil 2:1 bis 8:1 beträgt.

13. Zusammensetzung nach irgendeinem der vorhergehenden Ansprüche, die einen pH-Wert von 6,5 bis 8,5 aufweist.

14. Zusammensetzung nach irgendeinem der vorhergehenden Ansprüche, die einen pH-Wert von 7 bis 8 aufweist.

15. Zusammensetzung nach Anspruch 1, umfassend:
70 bis 90 Gew.-% Wasser;
0,75 bis 1,25 Gew.-% eines Tonanteils, wobei der Tonanteil Bentonit umfasst;
Polymerteilchen, die eine durchschnittliche Teilchengröße von weniger als 20 Mikron und einen Brechungsindex von 1,3 bis 1,4 aufweisen, wobei die Polymerteilchen einen vernetzten Polyester enthalten, der eine Mischung eines Polyesters von Adipinsäure und mit Isopropyltriethylsilan vernetztem Neopentylglycol, mit einem Copolymer von Vinylpyrrolidin und Vinylacetat gemischt, ist; und
ein zusätzliches Polymer, das ein gewichtsdurchschnittliches Molekulargewicht von 1000-750.000 aufweist.

16. Verwendung einer Zusammensetzung zum Behandeln der Haut unter den Augen, umfassend das topische Auftragen der Zusammensetzung auf die Haut unter den Augen, wobei die Zusammensetzung Folgendes umfasst:
Wasser;
0,75 bis 1,25 Gew.-% eines Tonanteils, wobei der Tonanteil Bentonit umfasst; und
Polymerteilchen, die eine durchschnittliche Teilchengröße von weniger als 20 Mikron und einen Brechungsindex von 1,3 bis 1,4 aufweisen, wobei die Polymerteilchen einen vernetzten Polyester enthalten, der eine Mischung eines Polyesters von Adipinsäure und mit Isopropyltriethylsilan vernetztem Neopentylglycol, mit einem Copolymer von Vinylpyrrolidin und Vinylacetat gemischt, ist;
wobei die Zusammensetzung vollständig frei von hydrophoben Verbindungen ist;
wobei eine hydrophobe Verbindung eine Verbindung ist, die einen hydrophoben Anteil enthält, der einer oder mehr der folgenden Kriterien entspricht: (a) eine Kohlenstoffkette von mindestens zehn Kohlenstoffen aufweist, wobei keiner der zehn Kohlenstoffe ein Carbonylkohlenstoff ist oder einen hydrophilen, direkt daran gebundenen Anteil aufweist; (b) zwei oder mehr Alkylsiloxygruppen aufweist und von hydrophilen Anteilen frei ist; oder (c) zwei oder mehr Oxypropylengruppen hintereinander aufweist, wobei die hydrophilen Anteile anionische, kationische oder zwitterionische Gruppen oder nichtionische Gruppen enthält, die polar sind.

## Revendications

1. Composition, comprenant :
de l'eau ;
0,75 % à 1,25 % en poids d'une portion d'argile, la portion d'argile comprenant de la bentonite ; et
des particules de polymère ayant une taille de particule moyenne inférieure à 20 microns et un indice de réfraction de 1,3 à 1,4, les particules de polymère comprenant un polyester réticulé qui est un mélange d'un polyester d'acide adipique et de néopentylglycol réticulé avec l'isopropyltriéthylsilane, mélangé avec un copolymère de vinylpyrrolidine et d'acétate de vinyle ; la composition étant totalement exempte de composés hydrophobes ;
dans laquelle un composé hydrophobe est un composé qui comprend un fragment hydrophobe satisfaisant à un ou plusieurs des critères suivants : (a) comporte une chaîne de carbone d'au moins dix carbones dans laquelle aucun des dix carbones est un carbone de carbonyle ou a un fragment hydrophile lié directement à celui-ci ; (b) comporte deux ou groupes alkylsiloxy ou plus et est exempt de fragments hydrophiles ; ou (c) comporte deux groupes oxypropylène ou plus en séquence, les fragments hydrophiles comprenant des groupes anioniques, cationiques ou zwitterioniques, ou des groupes non ioniques qui sont polaires.

2. Composition selon la revendication 1, dans laquelle la portion d'argile comprend au moins 20 % en poids de bentonite.

3. Composition selon la revendication 1 ou la revendication 2, dans laquelle la portion d'argile comprend en outre une hectorite synthétique.

4. Composition selon l'une quelconque des revendications précédentes, dans laquelle la portion d'argile comprend 70 à 80 % en poids d'hectorite synthétique et 20 à 30 % en poids de bentonite.

5. Composition selon l'une quelconque des revendications 1 à 3, dans laquelle la quantité de bentonite dans la portion d'argile est supérieure à 95 % en poids.

6. Composition selon la revendication 5, dans laquelle la portion d'argile est constituée de bentonite.

7. Composition selon l'une quelconque des revendications précédentes, comprenant 2 à 8 % en poids des particules de polymère.

8. Composition selon l'une quelconque des revendications précédentes, comprenant en outre un polymère supérieur ayant un poids moléculaire moyen en poids de 1000 à 750 000.

9. Composition selon l'une quelconque des revendications précédentes, dans laquelle les particules de polymère ont un indice de réfraction de 1,3 à 1,38.

10. Composition selon l'une quelconque des revendications précédentes, comprenant de 70 à 90 % en poids d'eau.

11. Composition selon l'une quelconque des revendications précédentes contenant moins de 2 % en poids de gommes de polysaccharide.

12. Composition selon l'une quelconque des revendications précédentes, dans laquelle le rapport en poids des particules de polymère à la portion d'argile est de 2:1 à 8:1.

13. Composition selon l'une quelconque des revendications précédentes ayant un pH de 6,5 à 8,5.

14. Composition selon l'une quelconque des revendications précédentes ayant un pH de 7 à 8.

15. Composition selon la revendication 1, comprenant :
70 % à 90 % en poids d'eau ;
0,75 % à 1,25 % en poids d'une portion d'argile, la portion d'argile comprenant de la bentonite ;
des particules de polymère ayant une taille de particule moyenne inférieure à 20 microns et un indice de réfraction de 1,3 à 1,4, les particules de polymère comprenant un polyester réticulé qui est un mélange d'un polyester d'acide adipique et de néopentylglycol réticulé avec l'isopropyltriéthylsilane, mélangé avec un copolymère de vinylpyrrolidine et d'acétate de vinyle ; et
un polymère supplémentaire ayant un poids moléculaire moyen en poids de 1000 à 750 000.

16. Utilisation d'une composition pour traitement de la peau sous les yeux sous les yeux comprenant l'application topique de la composition sur ladite peau sous les yeux, ladite composition comprenant :
de l'eau ;
0,75 % à 1,25 % en poids d'une portion d'argile, la portion d'argile comprenant de la bentonite ; et
des particules de polymère ayant une taille de particule moyenne inférieure à 20 microns et un indice de réfraction de 1,3 à 1,4, les particules de polymère comprenant un polyester réticulé qui est un mélange d'un polyester d'acide adipique et de néopentylglycol réticulé avec l'isopropyltriéthylsilane, mélangé avec un copolymère de vinylpyrrolidine et d'acétate de vinyle ; la composition étant totalement exempte de composés hydrophobes ;
dans laquelle un composé hydrophobe est un composé qui comprend un fragment hydrophobe satisfaisant à un ou plusieurs des critères suivants : (a) comporte une chaîne de carbone d'au moins dix carbones dans laquelle aucun des dix carbones est un carbone de carbonyle ou a un fragment hydrophile lié directement à celui-ci ; (b) comporte deux ou groupes alkylsiloxy ou plus et est exempt de fragments hydrophiles ; ou (c) comporte deux groupes oxypropylène ou plus en séquence, les fragments hydrophiles comprenant des groupes anioniques, cationiques ou zwitterioniques, ou des groupes non ioniques qui sont polaires.
